# EUROPÄISCHE PATENTSCHRIFT

(11) **EP 3 538 038 B1**
(45) Veröffentlichungstag und Bekanntmachung des Hinweises auf die Patenterteilung: **28.04.2021**
(21) Anmeldenummer: 17794733.0
(22) Anmeldetag: 08.11.2017
(51) Int. Cl.: A61F 5/02, A61F 13/00, A61F 13/04, A61F 13/06, A61F 5/01

(54) **SPANNBAND**
TENSIONING BAND
BANDE DE TENSION

(30) Priorität: 11.11.2016 DE 102016121657
(43) Veröffentlichungstag der Anmeldung: 18.09.2019
(73) Patentinhaber: Bauerfeind AG, 07937 Zeulenroda-Triebes (DE)
(72) Erfinder: BECK, André, 07646 Stadtroda (DE); ALBERT, Frank, 07937 Langenwolschendorf (DE); BAUERFEIND, Hans B., 07937 Zeulenroda-Triebes (DE)
(74) Vertreter: Wohlfahrt, Jan Günther
(86) Internationale Anmeldenummer: PCT/EP2017/078556
(87) Internationale Veröffentlichungsnummer: WO 2018/087122

(56) Entgegenhaltungen:
- EP-A1- 2 651 349
- WO-A2-2014/074645
- DE-A1-102012 009 214
- DE-A1-102014 012 654
- US-A1- 2009 082 707
- US-A1- 2009 118 655
- US-A1- 2014 276 318

## Beschreibung

Die vorliegende Erfindung betrifft ein Spannband, das in einem orthopädischen Hilfsmittel oder in einem Sporthilfsmittel verwendet werden kann, umfassend einen bandförmigen Grundkörper, der in Längsrichtung stauchbar ist und umfassend mindestens ein Zugseil, wobei das Zugseil mindestens einfach, bevorzugt mindestens zweifach entlang der Längsrichtung des Grundkörpers verläuft, und wobei das Zugseil bevorzugt an mindestens einem Ende des Grundkörpers umgelenkt werden kann. Die vorliegende Erfindung betrifft auch verschiedene Anwendungsmöglichkeiten des erfindungsgemäßen Spannbands, insbesondere auf dem Gebiet der medizinischen Hilfsmittel, orthopädischen Hilfsmittel oder Sporthilfsmittel.

In orthopädischen oder medizinischen Hilfsmitteln beziehungsweise Sporthilfsmitteln werden häufig Gurte und Bänder als Zuggurte oder Spanngurte eingesetzt. Beispielsweise beschreibt die DE 10 2010 054 579 A1 eine Orthese für die Dämpfung oder Begrenzung der Gelenkbewegung eines Extremitätengelenks, bei der eine Manschette und ein Wiederlager zu der Manschette über mindestens ein Zugband kraftschlüssig verbunden sind.

Inzwischen werden bislang verwendete Zuggurte oder Zugbänder durch Seile, insbesondere dünne Schnüre, also Seile mit einem geringen Durchmesser ersetzt, beispielsweise wird eine Gelenkbewegung durch ein Schnursystem einer solchen Orthese oder Bandage zu führen.

Leider haben Schnüre neben ihren Vorteilen (z.B. geringer Bauraum, hohe Flexibilität, geringer Kraftverlust durch Reibung), bedingt durch den geringen Durchmesser, den entscheidenden Nachteil des Einschneidens auf der Haut. Um das Einschneiden zu verringern muss die Fläche unter der Schnur vergrößert werden. Der heutige Stand der Technik sieht hierfür statische Platten- oder Schalenelemente vor, die sich, beim Verkürzen der Schnur, übereinander schieben und wie sie in der DE 20 2016 100 799 A1 beschreiben sind. Diese Elemente sind allerdings in ihrem Verstellweg sehr begrenzt. Die Platten- oder Schalenelemente müssen auch in einem aufwendigen Konfektions- und Montageprozess aus vielen Einzelteilen zusammengefügt und gegen ein Verkannten untereinander gesichert werden. Durch diesen Aufbau sind die Baugruppen recht steif und unflexibel. Die Schnurführung durch Tunnelbänder kann durch die Materialstauchung beim Spannen zum Faltenwurf der Tunnelbänder führen. Dadurch kann der Tragekomfort beeinträchtigt werden.

Dennoch sollen solche orthopädischen Hilfsmittel, medizinischen Hilfsmittel oder Sporthilfsmittel, insbesondere Orthesen oder Bandagen zukünftig immer schlanker, leichter und dünner werden, wobei die Wirksamkeit mindestens gleich bleiben soll.

Der vorliegenden Erfindung liegt daher das technische Problem zugrunde, die Nachteile aus dem Stand der Technik zu verbessern. Insbesondere sollen Vorrichtungen und Elemente bereitgestellt werden, die es erlauben, die vor allem in orthopädischen oder medizinischen Hilfsmitteln oder Sporthilfsmitteln, insbesondere Orthesen oder Bandagen, eingesetzten Bänder oder Seile, zu verbessern, wobei dabei insbesondere die Kraft der Seile so verteilt wird, dass die Seile nicht in die Haut des Trägers einschneiden. Ein weiteres Problem, dass der vorliegenden Erfindung zugrunde liegt, ist die Breitstellung einer Vorrichtung, die bei einer Umfangs- oder Längenveränderung eines oben genannten Hilfsmittels nicht zu einem Verschieben oder Einklemmen des darunter liegenden Hautgewebes führt.

Die vorliegende Erfindung löst das ihr zugrunde liegende technische Problem insbesondere durch ein Spannband nach Patentanspruch 1 und durch ein orthopädisches Hilfsmittels, medizinisches Hilfsmittel oder Sporthilfsmittel nach Patentanspruch 12.

Die vorliegende Erfindung löst das ihr zugrunde liegende technische Problem insbesondere durch ein Spannband, umfassend einen bandförmigen Grundkörper mit einer Längsrichtung, wobei der Grundkörper in Längsrichtung stauchbar und/oder dehnbar ist, und umfassend mindestens ein Zugseil, wobei das Zugseil mindestens einfach, bevorzugt mindestens zweifach entlang der Längsrichtung des Grundkörpers verläuft. Bevorzugt wird das Zugseil, insbesondere wenn es mindestens zweifach entlang der Längsrichtung des Grundkörpers verläuft, an mindestens einem Ende des Grundkörpers umgelenkt. Insbesondere wenn das Zugseil einfach entlang der Längsrichtung des Grundkörpers verläuft, ist das Zugseil bevorzugt an einem Ende des Grundkörpers mit diesem verbunden, insbesondere fest verbunden.

Ein solches Spannband erlaubt in vorteilhafter Weise durch seine konstruktiven Eigenschaften, also Form, Struktur und Materialeigenschaften ein formschlüssiges flexibles Anlegen an einen beliebigen Untergrund. Durch den Verzicht auf feste statische Elemente wie zum Beispiel interne Führungselemente in Längs oder Querrichtung weist das Spannband in allen Achsen sowie in der Torsion ein flexibles Verhalten auf. Somit ist es in vorteilhafter Weise möglich die auftretenden Kräfte flexibel über die Anlageflächen abzuleiten.

Es zeigte sich, dass die erfindungsgemäßen Spannbänder eine Verwendung eines Seils, insbesondere einer Schnur in einem medizinischen Hilfsmittel, orthopädischen Hilfsmittel oder Sporthilfsmittel, insbesondere in einer Orthese oder Bandage, ermöglichen, ohne dass die Seile, insbesondere Schnüre in die Haut des Trägers des Hilfsmittels einschneiden. Dies wird insbesondere auch dadurch erreicht, dass das Grundelement großflächig die Haut des Trägers abdeckt und einen direkten Kontakt des Seils mit der Haut und somit ein Einschneiden in die Haut durch die großflächige Kraftverteilung verhindert.

Bevorzugt ist das Zugseil beweglich im Grundkörper gelagert.

Bevorzugt verläuft das Zugseil zweifach bis fünffach entlang der Längsrichtung des Grundkörpers, insbesondere zweifach bis vierfach entlang der Längsrichtung des Grundkörpers.

Bevorzugt ist das Zugseil bei einem mindestens zweifachen Verlauf entlang der Längsrichtung des Grundkörpers als Flaschenzug ausgestaltet. Bevorzugt ist das Zugseil als Flaschenzug ausgebildet. Bevorzugt ist dabei das Zugseil an einem Ende des Grundkörpers befestigt ist. Die Ausführung als Flaschenzug hat den Vorteil, dass der Kraftaufwand zum Zusammenziehen des Grundkörpers geringer ist.

Das Flaschenzugsystem ermöglicht einen axial verlaufenden Kraft-Fluss entlang der neutralen Faser, die die ideale Biegelinie bildet. Dabei spielt es keine Rolle, in welcher Lage sich das Spannband befindet. Als neutrale Faser, auch Nulllinie genannt, bezeichnet man in der Festigkeitslehre diejenige Faser oder Schicht eines Querschnitts eines länglichen Körpers, deren Länge sich bei Verdrehen bzw. Biegen nicht ändert. Dort verursacht die Beanspruchung keine Zug-, Druck-, oder Scherspannung, die neutrale Faser ist kräftefrei. Sie verläuft durch den geometrischen Schwerpunkt der Querschnittsfläche des Balkens, wenn dieser gerade ist.

Durch eine bevorzugte doppelte Umlenkung überträgt sich die eingeleitete Kraft der Schnur auch bei verschiedenen Lagen des Spannbands gleichmäßig auf den Körper.

Bevorzugt verläuft das Zugseil dreifach entlang der Längsrichtung des Grundkörpers.

Das erfindungsgemäße Spannband lässt sich in den verschiedensten Bereichen einsetzen, insbesondere bei orthopädischen Hilfsmitteln, medizinischen Hilfsmitteln oder Sporthilfsmitteln. Bevorzugt ist das Spannband Bestandteil eines orthopädischen Hilfsmittels eines medizinischen Hilfsmittels oder eines Sporthilfsmittel.

Bevorzugt ist das Spannband zum Spannen eines Gurtes um einen Körperteil, beispielsweise ein Bein, ein Arm oder der Oberkörper, geeignet. Das Spannband kann aber auch als Gurt verendet werden, der um einen Körperteil gespannt ist. Mit dem Spannband können aber auch andere Elemente, beispielsweise Teilbereiche einer Orthese oder Bandage gespannt werden. Das Spannband kann in vorteilhafter Weise nicht nur einen Gurt oder eine Schnur ersetzen, sondern es kann alternativ auch an einem Gurt angesetzt sein um den Gurt spannen oder entspannen zu können.

Bevorzugt ist der Grundkörper als Netzband oder Gitterband ausgestaltet. Ein solches Netzband oder Gitterband hat zusätzlich den Vorteil, dass es besonders leicht und atmungsaktiv ist, insbesondere im Vergleich zu einem herkömmlichen Zuggurt.

Bevorzugt hat das Netzband oder das Gitterband eine karoförmige Struktur. Diese ist besonders gut komprimierbar.

Bei dem Grundkörper handelt es sich also erfindungsgemäß bevorzugt um einen gitterartig beziehungsweise netzartig aufgebauten Grundkörper. Durch die Gitterstruktur lässt sich der Grundkörper in vorteilhafter durch das Spannen des Spannbands zusammenziehen und ein Entspannen des Spannbands erlaubt das Auseinanderziehen des Grundkörpers.

Bevorzugt verläuft das Zugseil mindestens einfach durch das Netzband oder Gitterband. Beispielsweise kann das Netzband oder Gitterband durch Auslassungen eine Rinne bilden, in der das Zugseil verläuft. Insbesondere kann vorgesehen sein, dass das Netzband oder Gitterband teilweise auf der Oberseite und teilweise auf der Unterseite Auslassungen aufweist, insbesondere dass sich die Auslassungen auf der Oberseite und auf der Unterseite des Netzbands oder Gitterbands abwechseln. Dadurch wird das bewegliche Zugseil im Netzband oder Gitterband gehalten. Alternativ kann das Netzband oder Gitterband Löcher in Längsrichtung aufweisen, beispielsweise Bohrungen, durch die das Zugseil hindurchgezogen ist.

Bevorzugt wird das Netzband oder Gitterband aus einem flexiblen Material, insbesondere Kunststoff gebildet.

Bevorzugt wird das Netzband oder Gitterband aus einem flexiblen Material, insbesondere Kunststoff gebildet, das eine Vielzahl von Ausnehmungen aufweist.

Bevorzugt wird das Netzband oder Gitterband aus einem flexiblen Material, insbesondere Kunststoff gebildet, das eine Vielzahl von rautenförmigen Ausnehmungen aufweist. Dem Fachmann sind geeignete flexible Materialien bekannt, bis hin zu gummiartigen Kunststoffen. Bevorzugt wird für den Grundkörper ein Material mit geringen Reibungseigenschaften gewählt.

Der Grundkörper kann auch aus mehreren verschiedenen Materialien bestehen. Beispielsweise ist eine 2-Komponententechnik möglich, bei der der Grundkörper im Spritzgussverfahren hergestellt wird. Daher können in vorteilhafter Weise an allen Stellen, an denen die Schnur durch Umlenkung hohe Reibung erfährt, Materialien, beispielsweise Kunststoffe, mit geringem Reibungskoeffizienten eingesetzt werden, während in den anderen Bereichen andere Kunststoffe mit anderen Eigenschaften eingesetzt werden können. Das Material des Grundkörpers kann auch elastisch sein oder im Wesentlichen unelastisch.

In einer Ausführungsform ist dem Grundkörper an einem seiner Längsrichtungsenden ein Endelement zugeordnet, das eine Befestigungsvorrichtung für das Zugseil aufweist.

In einer Ausführungsform ist dem Grundkörper an dessen Längsrichtungsenden jeweils ein Endelement zugeordnet, das eine Befestigungsvorrichtung und/oder eine Umlenkvorrichtung für das Zugseil aufweist. Damit kann das Zugseil an mindestens einem Endelement befestigt sein und zumindest durch das andere Endelement umgelenkt werden, so dass es im Grundkörper verläuft und mittelbar mit dem Grundkörper verbunden ist, aber nicht unmittelbar und direkt mit am Grundkörper befestigt ist. Die Endelemente können fest oder koppelbar mit einem Gurt verbunden sein. Die Endelemente können auch als Befestigungsmittel für das Spannband an einem orthopädischen Hilfsmittel, medizinischen Hilfsmittel oder Sporthilfsmittel verwendet werden.

In einer alternativen Ausführungsform ist dem Grundkörper an einem Längsrichtungsende ein Element zum Spannen und Aufrollen des Zugseils zugeordnet. Das Element zum Spannen und Aufrollen kann sich beispielsweise auf oder in einem Endelement befinden oder an das Endelement angesetzt sein. Dem Fachmann sind geeignete Elemente zum Spannen und Aufrollen eines Zugseils bekannt, beispielsweise Drehknöpfe mit einer arretierbaren Spule.

In einer bevorzugten Ausführungsform ist das Zugseil eine Schnur, also ein Seil mit einem dünnen Durchmesser. Dem Fachmann sind geeignete Schnüre und geeignete Materialen für solche Schnüre bekannt.

Das Zugseil kann aus einem Faden, Kabel oder ähnlichem bestehen. Das Zugseil überträgt die auftretenden Zugkräfte in Querrichtung des Grundkörpers und wandelt diese in Flächenpressung um. Damit wird in vorteilhafter Weise ein exaktes Aufliegen auch auf stark konturierten Oberflächen ermöglicht wird, ohne die Funktion zu beeinträchtigen.

Die vorliegende Erfindung betrifft auch Produkte, die ein erfindungsgemäßes Spannband aufweisen. Insbesondere betrifft die Erfindung ein orthopädisches Hilfsmittel, medizinisches Hilfsmittel oder Sporthilfsmittel, umfassend ein erfindungsgemäßes Spannband.

Bevorzugt ist das Spannband in oder an einem orthopädischen oder medizinischen Hilfsmittel oder Sporthilfsmittel, insbesondere in oder an einer Orthese oder Bandage befestigt, insbesondere Bestandteil eines orthopädischen oder medizinischen Hilfsmittels oder Sporthilfsmittels, insbesondere einer Orthese oder Bandage.

Bevorzugt ist das orthopädische Hilfsmittel eine Orthese.

Bei der Orthese kann es sich beispielsweise um eine Armorthese, Beinorthese oder Rückenorthese handeln. Bevorzugt handelt es sich um eine Orthese für Extremitäten. Beispielsweise kann es sich um eine Orthese handeln, die eine stützende oder bewegungsgleitende Funktion ausübt, insbesondere eine Orthese zur Dämpfung oder Begrenzung der Beugung oder Streckbewegung eines Extremitätengelenks, wie Ellenbogengelenk oder Kniegelenk. Eine solche Orthese ist beispielsweise aus der DE 10 2010 054 579 A1 bekannt. Ein Fachmann kann ohne weiteres die dort gezeigten Zugbänder durch Seile, insbesondere Schnüre und den dort gezeigten Druckeinleitungsabschnitt durch ein erfindungsgemäßes Spannband ersetzen. Die vorliegende Erfindung betrifft daher bevorzugt auch eine dementsprechend modifizierte Orthese, wie sie in der DE 10 2010 054 579 A1 beschrieben ist. Der Gegenstand der DE 10 2010 054 579 A1 ist daher in die vorliegende Offenbarung mit einbezogen.

Bevorzugt ist ein orthopädisches Hilfsmittel, medizinisches Hilfsmittel oder Sporthilfsmittel, bei dem das Spannband zum Umgreifen eines Körperteils, das einen Zirkelschluss des Spannbands erlaubt, ausgestaltet ist.

Eine bevorzugte Ausführungsform einer erfindungsgemäßen Orthese ist eine Orthese zur Dämpfung oder Begrenzung der Gelenkbewegung eines Extremitätengelenks, aufweisend eine die Extremität unterhalb des Gelenks umgreifende Manschette, die mit einem an der Extremität oberhalb des Gelenks anlegbaren Widerlager gekoppelt ist, wobei sich von dem Widerlager zu der Manschette das Zugseil erstreckt, das mit Widerlager und Manschette kraftschlüssig verbunden ist, wobei der Manschette der Grundkörper des Spannbands zugeordnet ist und das Spannband als Druckeinleitungsabschnitt der Manschette ausgebildet ist, und wobei das Zugseil derart ausgestaltet ist, dass es im angelegten Zustand der Orthese durch die Gelenkbewegung der Extremität spannbar ist, um dabei über das Spannband Kompression auf einen darunter liegenden Weichteilbereich der Extremität auszuüben, um die Gelenkbewegung zu dämpfen oder zu begrenzen.

Bevorzugt kann das Zugseil im Bereich des Widerlagers durch ein Aufrollelement gespannt werden.

In einer bevorzugten Ausführungsform verläuft bei der Orthese das Zugseil zweifach und überkreuzt von dem Widerlager zu dem Druckeinleitungsabschnitt der Manschette und wird an dem Druckeinleitungsabschnitt durch das erfindungsgemäße Umlenkelement umgelenkt.

In einer bevorzugten Ausführungsform ist das Widerlager als zweite Manschette ausgebildet.

In einer bevorzugten Ausführungsform handelt es sich bei der ersten Manschette und/oder bei der zweiten Manschette um flexible Manschettengurte, die bevorzugt mittels Klettverschluss geöffnet und angezogen werden können.

In einer bevorzugten Ausführungsform sind Manschette und Widerlager auf ein textiles Gestrick aufgerüstet.

In einer bevorzugten Ausführungsform kann das Zugseil im Bereich des Widerlagers durch ein Aufrollelement gespannt werden. Es wird also in dieser bevorzugten Ausführungsform das Zugseil durch das Aufrollelement gespannt, wodurch im Bereich der ersten Manschette der Grundkörper des Spannbands zusammengezogen wird, so dass das Spannband die Extremität stärker umgreift.

In einer weiteren bevorzugten Ausführungsform ist das Spannband nicht zirkulär umschließend verwendet, sondern überspannt nur einen Teilabschnitt einer Kreisbahn oder einer Gerade. Der nicht vom spannband überspannte Abschnitt der Kreisbahn oder der Gerade kann wahlweise elastisch oder unelastisch ausgeführt sein.

Beispielsweise kann das spannband dazu verwendet werden um nur einen Teilbereich mit Druck zu belegen.

In einer weiteren Ausführungsform kann das Spannband auch linear statt zirkulär geführt sein. Diese Ausführungsform kann beispielweise bei einer Rückenorthese, insbesondere bei den Gurten einer Rückenorthese verwendet werden. Dabei kann das Spannband von einem Bereich eines elastischen oder unelastischen Gestricks unterbrochen sein.

Bevorzugte Ausführungsformen ergeben sich auch aus den Unteransprüchen.

Die vorliegende Erfindung wird anhand der Figuren näher und beispielhaft erläutert, wobei die Figuren und dazugehörige Erläuterung nicht einschränkend zu verstehen sind.

Es zeigen
- Figur 1: ein erfindungsgemäßes Spannband in entspanntem Zustand;
- Figur 2: das erfindungsgemäße Spannband von Figur 1 in gespanntem Zustand;
- Figur 3: eine alternative Ausführungsform des erfindungsgemäßen Spannbands;
- Figur 4: ein erfindungsgemäßes Spannband mit Aufrollelement und Gurt;
- Figur 5: ein orthopädisches Hilfsmittel mit einem erfindungsgemäßen Spannband;
- Figur 6: ein weiteres orthopädisches Hilfsmittel mit einem erfindungsgemäßen Spannband.

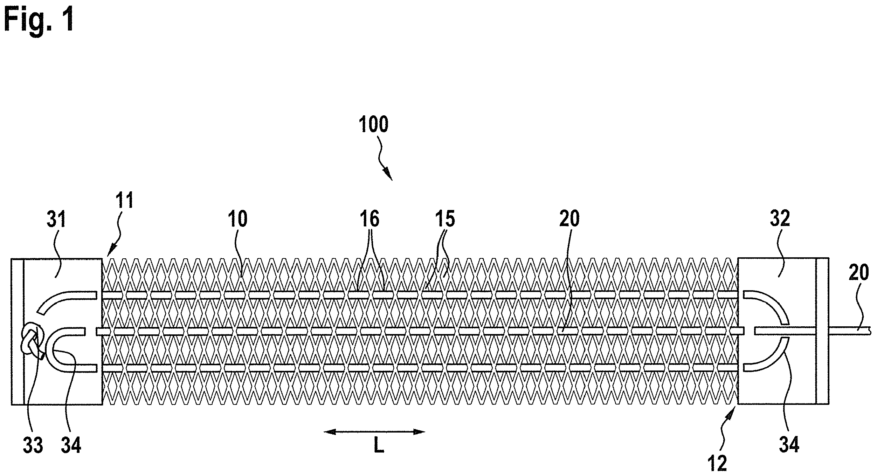

Figur 1 zeigt ein erfindungsgemäßes Spannband (100) in entspanntem Zustand. Das Spannband (100) hat einen bandförmigen Grundkörper (10) in der Ausgestaltung als Netzband oder Gitterband. In dem bandförmigen Grundkörper (10) verläuft ein Zugseil (20), wobei das Zugseil (20) dreifach entlang der Längsrichtung (L) des bandförmigen Grundkörpers (10) geführt ist. Der bandförmige Grundkörper (10) besteht aus einem flexiblen Material und weist als Netzband oder Gitterband eine Vielzahl von rautenförmigen Ausnehmungen (15) auf. Darüber hinaus weist der bandförmige Grundkörper (10) Einschnitte (16) auf, in denen das Zugseil (20) beweglich gelagert ist. Die Einschnitte (16) befinden sich abwechselnd auf der Oberseite und Unterseite des Grundkörpers (10), wodurch das Zugseil (20) dem Grundkörper (10) zugeordnet bleibt, aber dennoch in Längsrichtung (L) beweglich und ziehbar ist. Dem bandförmigen Grundkörper (10) ist an seinen beiden Längsrichtungsenden (11,12) jeweils ein Endelement (31,32) zugeordnet. Das Zugseil (20) ist am Endelement (31) über eine Befestigungsvorrichtung (33) mit dem bandförmigen Grundkörper (10) indirekt verbunden. Das Befestigungselement ist hier als Steg (33) ausgebildet, durch den das Zugseil (20) hindurchgezogen ist und durch einen Knoten gesichert ist. Am gegenüberliegenden Endelement (32) ist das Zugseil (20) nicht befestigt, sondern tritt aus dem Endelement (32) heraus und kann dort gezogen werden.

Beide Endelemente (31,32) weisen Umlenkvorrichtungen (34) auf, mit denen das Zugseil (20) umgelenkt wird, sodass es als Flaschenzug dreifach durch den bandförmigen Grundkörper (10) hindurchläuft. Die Umlenkvorrichtungen (34) können beispielsweise als grabenartige Vertiefungen in den Endelementen (31,32) ausgebildet sein, in denen das Zugseil (20) verläuft und die die Verlaufsrichtung des Zugseils (20) vorgeben.

Wird das Zugseil (20) am herausragenden Ende im Bereich des Endelements (32) gezogen, so zieht sich dadurch der bandförmige Grundkörper (10) in Längsrichtung (11) ziehharmonikaförmig zusammen und das Spannband (100) wird gespannt.

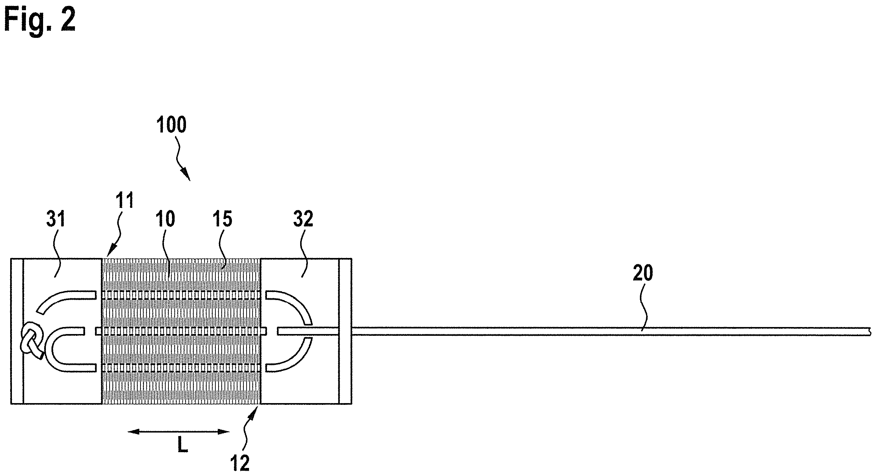

Figur 2 zeigt das Spannband (100) in gespanntem Zustand. Das Zugseil (20) wurde herausgezogen, sodass der bandförmige Grundkörper (10) sich in Längsrichtung (L) zusammengezogen hat, wodurch sich die rautenförmigen Ausnehmungen (15) verkleinern und die beiden Längsenden (11,12) mit den Endelementen (31,32) zusammengezogen sind und näher beieinander liegen.

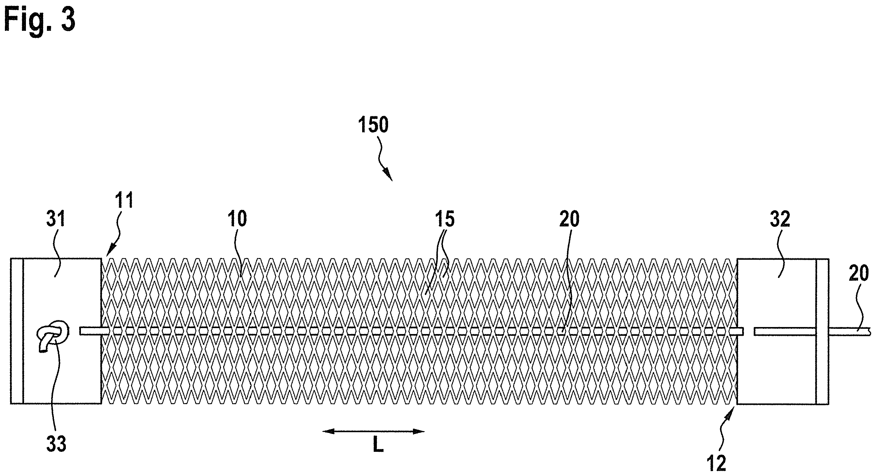

Figur 3 zeigt eine alternative Ausführung des erfindungsgemäßen Spannbands (150). In dieser Ausführungsform ist das Zugseil (20) einfach durch den bandförmigen Grundkörper (10) in Längsrichtung (L) geführt. Auch ist das Zugseil (20) nicht in Vertiefungen des bandförmigen Grundkörpers (10) eingelegt, sondern durch Löcher im bandförmigen Grundkörper (10) hindurchgezogen. Durch die einfache Zugführung sind keine Umlenkelemente notwendig. Zu sehen sind aber wieder die beiden Längsrichtungsenden (11,12) mit den dortigen Endelementen (31,32), wobei das Zugseil (20) wieder über einen Steg und einen Knoten als Befestigungsvorrichtung (33) mit dem Endelement (31) befestigt ist. Die Funktion des gezeigten Spannband (150) ist die gleiche, wie die des Spannbands (100) aus Figur 1, wobei in der vorliegenden Ausführungsform kein Flaschenzugeffekt auftritt.

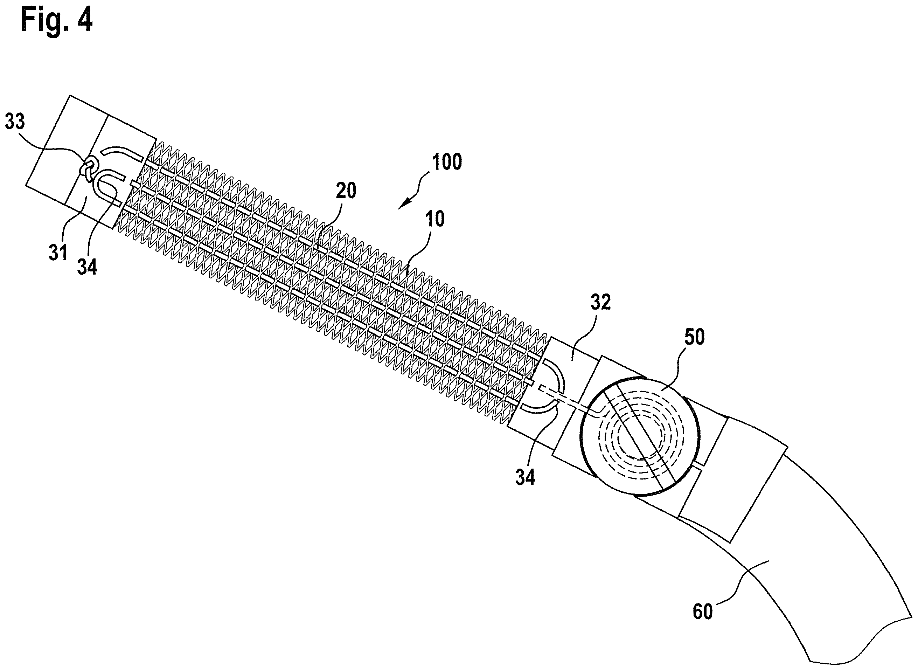

Figur 4 zeigt das erfindungsgemäße Spannband (100) aus Figur 1, mit einem Aufrollelement (50) und einem daran befestigten Gurt (60). Das Spannband (100) weist wieder die schon in Figur 1 gezeigten Elemente auf, nämlich den bandförmigen Grundkörper (10), durch den das Zugseil (20) verläuft, wobei das Zugseil (20) an einem Endelement (31) über eine Steg/Knotenkombination (33) befestigt ist und an den beiden Endelementen (31,32) jeweils einmal mittels einer Umlenkvorrichtung (34) umgelenkt wird. An das Endelement (32), aus dem das Zugseil (20) heraustritt, ist ein Aufrollelement (50) in Form einer drehbaren Spule befestigt, mit dem das Zugseil (20) zum Spannen des Spannelements (100) aufgerollt werden kann. An der anderen Seite des Aufrollelements (50) ist ein Gurt (60) befestigt. Das Spannelement (100) kann als Teilstück des Gurtes (60) angesehen werden, sodass die Gesamtgurtlänge durch Aufrollen des Zugseils (20) auf das Aufrollelement (50) und das dadurch bewirkte Zusammenziehen des bandförmigen Grundkörpers (10) verkürzt wird.

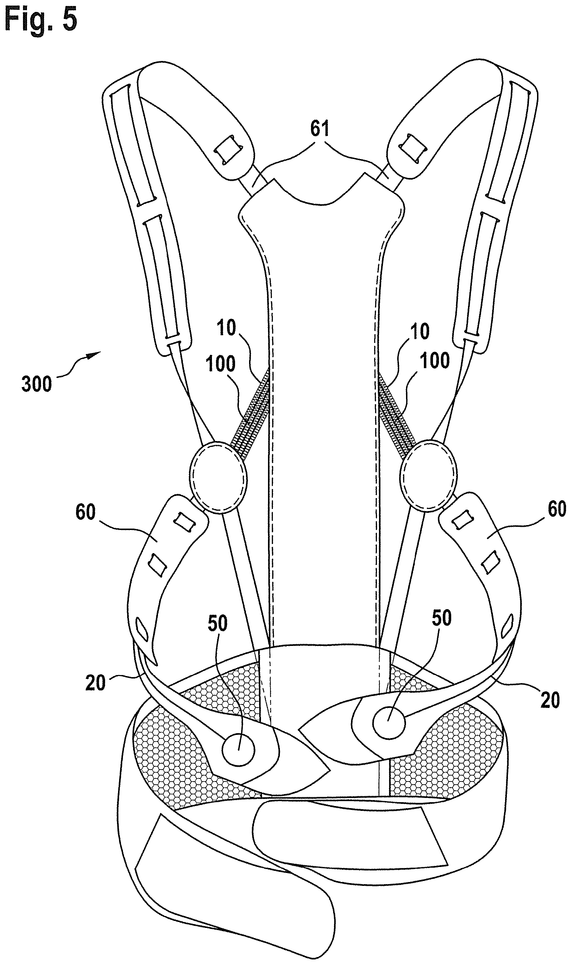

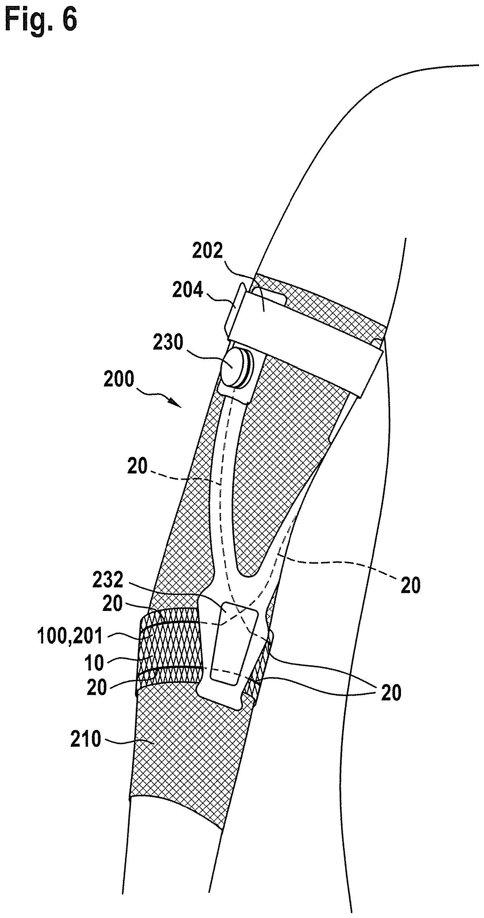

Die Figuren 5 und 6 zeigen Verwendungsbeispiele des erfindungsgemäßen Spannbands (100) in orthopädischen Hilfsmitteln.

Figur 5 zeigt eine Rückenorthese (300). Diese wird mit Schultergurten (60,61) am Körper getragen. Damit die Rückenorthese (300) auf den Rücken wirken kann, müssen die Schultergurte (60,61) gespannt werden. Dazu sind die Gurte (60,61) mit zwei erfindungsgemäßen Spannbändern (100) versehen. Die Spannbänder weisen wieder jeweils einen bandförmigen Grundkörper (10) auf, dem wie in Figur 1, ein Zugseil (20) zugeordnet ist. Das Zugseil tritt aus dem bandförmigen Grundkörper (10) aus, und verläuft entlang eines Gurtabschnitts (60) jeweils eines der Gurte (60,61). Am Ende des Gurtabschnitts (60) befindet sich ein Aufrollelement (50), mit dem das Zugseil (20) aufgerollt werden kann. Durch das Aufrollen des Zugseils (20) wird der bandförmige Grundkörper (10) zusammengezogen, wodurch die Schultergurte (60,61) angespannt und an den Körper angepresst werden, sodass die Rückenorthese (300) den gewünschten Druck auf den Rücken ausüben kann. Um die Schultergurte (60,61) beim Ablegen der Rückenorthese (300) wieder zu entspannen kann beispielsweise das Aufrollelement (50) entkoppelt werden, wodurch das Zugseil (20) wieder von diesem abgerollt werden kann. Durch die Eigensteifigkeit des bandförmigen Grundkörpers (10) und weiterer fakultativer konstruktiver Eigenschaften kann der bandförmige Grundkörper (10) in seine entspannte Ursprungsform zurückkehren, wodurch ein einfaches Repositionieren und/oder Ablegen der Orthese (300) ermöglicht wird.

Figur 6 zeigt eine Armorthese (200) mit einem erfindungsgemäßen Spannband (100). Das Spannband (100) bildet eine Manschette (201), die zirkulär um den Unterarm liegt. Die Manschette (201) ist mit einem den Oberarm umschließenden Widerlager (202) über ein Kopplungselement (232) gekoppelt. Das Kopplungselement (232) dient dem Umlenken des Zugseils (20) in Richtung Widerlager (202). Der Fachmann kann aber auch alternative Umlenkungselemente vorsehen. Beispielsweise kann ein wie in den Figuren 1 bis 3 gezeigtes Spannband komplett um den Unterarm herumreichen, wobei die beiden Enden des Spannbands über ein Endelement miteinander verbunden sind und das Endelement zum Umlenken des Zugseils in Richtung Widerlager dient. Das Widerlager (202) weist ein Aufrollelement (230) auf, in das das Zugseil (20) des Spannbands (100) aufgerollt werden kann. Dadurch kann eine Grundspannung erzeugt werden, durch die der bandförmige Grundköper (10) zusammengezogen wird und einen Grunddruck auf das darunterliegende als Maschenware ausgefertigte Orthesenmaterial (210) und den Unterarm ausübt.

Wird der Arm durchgestreckt, so verlängert sich der Weg, den das Zugseil (20) von der Manschette (201) zum Widerlager (202) überspannt. Dadurch wird das Zugseil (20) noch mehr angespannt, wodurch sich der bandförmige Grundkörper (10) noch mehr zusammenzieht und noch mehr Druck auf den Unterarm ausgeübt wird. Der nun auftretende Druck ist so stark, dass ein weiteres Anspannen und somit Überspannen des Arms verhindert wird. Dies ist beispielsweise im Training von Wurfsportarten hilfreich.

## Patentansprüche

1. Spannband (100,150), das in einem orthopädischen Hilfsmittel oder in einem Sporthilfsmittel verwendet werden kann, umfassend einen bandförmigen Grundkörper (10) mit einer Längsrichtung (L), wobei der Grundkörper (10) in Längsrichtung (L) stauchbar ist, und umfassend mindestens ein Zugseil (20), wobei das Zugseil (20) mindestens einfach entlang der Längsrichtung (L) des Grundkörpers (10) verläuft, wobei der Grundkörper als Netzband oder Gitterband ausgestaltet ist.

2. Spannband (100,150) nach Anspruch 1, wobei das Spannband (100) zum Spannen eines Gurtes (200) um einen Körperteil geeignet ist.

3. Spannband (100) nach einem der vorhergehenden Ansprüche, wobei das Zugseil (20) mindestens zweifach entlang der Längsrichtung (L) des Grundkörpers (10) verläuft.

4. Spannband (100) nach Anspruch 3, wobei das Zugseil (20) an mindestens einem Ende (11,12) des Grundkörpers (10) umgelenkt wird und/oder wobei das Zugseil (20) als Flaschenzug ausgebildet ist.

5. Spannband (100,150) nach einem der vorhergehenden Ansprüche, wobei das Spannband (100,150) Bestandteil eines orthopädischen Hilfsmittels (200), eines medizinischen Hilfsmittels oder eines Sporthilfsmittel ist.

6. Spannband (100,150) nach einem der vorhergehenden Ansprüche, wobei das Zugseil (20) beweglich im Grundkörper (10) gelagert ist und/oder wobei das Zugseil (20) an einem Ende (11) des Grundkörpers (10) befestigt ist.

7. Spannband (100,150) nach einem der vorhergehenden Ansprüche, wobei das Netzband oder Gitterband aus Kunststoff gebildet wird und/oder wobei das Netzband oder Gitterband eine Vielzahl von Ausnehmungen (15) aufweist.

8. Spannband (100) nach einem der vorhergehenden Ansprüche, wobei dem Grundkörper (10) an dessen Längsrichtungsenden (11,12) jeweils ein Endelement (31,32) zugeordnet ist, das eine Befestigungsvorrichtung (33) und/oder eine Umlenkvorrichtung (34) für das Zugseil (20) aufweist.

9. Spannband (100,150) nach einem der vorhergehenden Ansprüche, wobei dem Grundkörper (10) an einem Längsrichtungsende (11,12) ein Aufrollelement (50) zum Spannen und Aufrollen des Zugseils (20) zugeordnet ist.

10. Orthopädisches Hilfsmittel, medizinisches Hilfsmittel oder Sporthilfsmittel, umfassend ein Spannband (100,150) nach einem der vorhergehenden Schutzansprüche.

11. Orthopädisches Hilfsmittel nach Anspruch 10, wobei das orthopädische Hilfsmittel eine Orthese (200,300) ist.

12. Orthopädisches Hilfsmittel nach Anspruch 10 oder nach Anspruch 11, wobei das Spannband (100,150) zum Umgreifen eines Körperteils, das einen Zirkelschluss des Spannbands (100,150) erlaubt, ausgestaltet ist.

13. Orthese nach Anspruch 11 bis Anspruch 12, wobei die Orthese (200) zur Dämpfung oder Begrenzung der Gelenkbewegung eines Extremitätengelenks, aufweisend eine die Extremität unterhalb des Gelenks umgreifende Manschette (201), die mit einem an der Extremität oberhalb des Gelenks anlegbaren Widerlager (202) gekoppelt ist, wobei sich von dem Widerlager (202) zu der Manschette (201) das Zugseil (20) erstreckt, das mit Widerlager (202) und Manschette (201) kraftschlüssig verbunden ist, wobei der Manschette der Grundkörper (10) des Spannbands (100) zugeordnet ist und das Spannband (100) als Druckeinleitungsabschnitt der Manschette (201) ausgebildet ist, und wobei das Zugseil (20) derart ausgestaltet ist, dass es im angelegten Zustand der Orthese (200) durch die Gelenkbewegung der Extremität spannbar ist, um dabei über das Spannband (100,150) Kompression auf einen darunter liegenden Weichteilbereich der Extremität auszuüben, um die Gelenkbewegung zu dämpfen oder zu begrenzen.

14. Orthese nach Anspruch 13, wobei das Zugseil (20) im Bereich des Widerlagers (202) durch ein Aufrollelement (230) gespannt werden kann.

## Claims

1. A tensioning strap (100, 150), which can be used in an orthopedic aid or in a sports aid comprising a strap-shaped base body (10) with a longitudinal direction (L), wherein the base body (10) is compressible in the longitudinal direction (L), and comprising at least one pull cable (20), wherein the pull cable (20) runs at least once along the longitudinal direction (L) of the base body (10), wherein the base body is designed as a netted strap or a mesh strap.

2. The tensioning strap (100, 150) according to claim 1, wherein the tensioning strap (100) is suitable for tensioning a belt (200) around a part of the body.

3. The tensioning strap (100) according to one of the preceding claims, wherein the pull cable (20) runs at least twice along the longitudinal direction (L) of the base body (10).

4. The tensioning strap (100) according to claim 3, wherein the pull cable (20) is deflected at at least one end (11, 12) of the base body (10) and/or wherein the pull cable (20) is formed as a pulley block.

5. The tensioning strap (100, 150) according to one of the preceding claims, wherein the tensioning strap (100, 150) is a component of an orthopedic aid (200), a medical aid, or a sports aid.

6. The tensioning strap (100, 150) according to one of the preceding claims, wherein the pull cable (20) is movably mounted in the base body (10) and/or wherein the pull cable (20) is fastened at one end (11) of the base body (10).

7. The tensioning strap (100, 150) according to one of the preceding claims, wherein the netted strap or mesh strap is formed from a synthetic material, and/or wherein the netted strap or mesh strap has a multiple number of recesses (15).

8. The tensioning strap (100) according to one of the preceding claims, wherein a respective end element (31, 32), which has a fastening device (33) and/or a deflecting device (34) for the pull cable (20), is assigned to the base body (10) at its longitudinal direction ends (11, 12).

9. The tensioning strap (100, 150) according to one of the preceding claims, wherein a roll-up element (50) for tensioning and rolling up the pull cable (20) is assigned to the base body (10) at one longitudinal direction end (11, 12).

10. An orthopedic aid, medical aid, or sports aid comprising a tensioning strap (100, 150) according to one of the preceding claims for protection.

11. The orthopedic aid according to claim 10, wherein the orthopedic aid is an orthosis (200, 300).

12. The orthopedic aid according to claim 10 or according to claim 11, wherein the tensioning strap (100, 150) is designed to encompass a part of the body, which permits a circular closure of the tensioning strap (100, 150).

13. The orthosis according to claim 11 through claim 12, wherein the orthosis (200) for cushioning or limiting the joint movement of an extremity joint, having a cuff (201) encompassing the extremity below the joint, which is coupled to an abutment (202) that can be applied to the extremity above the joint, wherein the pull cable (20) extends from the abutment (202) to the cuff (201), which is connected in a force-fitting manner to abutment (202) and cuff (201), wherein the base body (10) of the tensioning strap (100) is assigned to the cuff and the tensioning strap (100) is formed as a pressure initiation section of the cuff (201), and wherein the pull cable (20) is formed in such a manner that, in the applied state of the orthosis (200), it can be tensioned by the joint movement of the extremity, in order to thereby, via the tensioning strap (100, 150), exert compression on an underlying soft-tissue region of the extremity, in order to cushion or limit the joint movement.

14. The orthosis according to claim 13, wherein the pull cable (20) can be tensioned in the area of the abutment (202) by a roll-up element (230).

## Revendications

1. Bande de compression (100, 150), qui peut être utilisée dans un auxiliaire orthopédique ou dans un auxiliaire du sport, comprenant un corps de base (10) en forme de bande avec un sens longitudinal (L), dans laquelle le corps de base (10) est compressible dans le sens longitudinal (L), et comprenant au moins une corde de traction (20), dans laquelle la corde de traction (20) s'étend au moins simplement le long du sens longitudinal (L) du corps de base (10), dans laquelle le corps de base est configuré en tant que bande à maillage ou bande en treillis.

2. Bande de compression (100, 150) selon la revendication 1, dans laquelle la bande de compression (100) est adaptée pour tendre une sangle (200) autour d'une partie du corps.

3. Bande de compression (100) selon l'une quelconque des revendications précédentes, dans laquelle la corde de traction (20) s'étend au moins deux fois le long du sens longitudinal (L) du corps de base (10).

4. Bande de compression (100) selon la revendication 3, dans laquelle la corde de traction (20) est renvoyée sur au moins une extrémité (11, 12) du corps de base (10) et/ou dans laquelle la corde de traction (20) est réalisée en tant que poulie.

5. Bande de compression (100, 150) selon l'une quelconque des revendications précédentes, dans laquelle la bande de compression (100, 150) fait partie intégrante d'un auxiliaire orthopédique (200) d'un auxiliaire médical ou d'un auxiliaire du sport.

6. Bande de compression (100, 150) selon l'une quelconque des revendications précédentes, dans laquelle la corde de traction (20) est montée de manière mobile dans le corps de base (10) et/ou dans laquelle la corde de traction (20) est fixée sur une extrémité (11) du corps de base (10).

7. Bande de compression (100, 150) selon l'une quelconque des revendications précédentes, dans laquelle la bande à maillage ou la bande en treillis est formée à partir de matière plastique et/ou dans laquelle la bande à maillage ou la bande en treillis présente une pluralité d'évidements (15).

8. Bande de compression (100) selon l'une quelconque des revendications précédentes, dans laquelle est associé au corps de base (10) au niveau de ses extrémités de sens longitudinal (11, 12) respectivement un élément d'extrémité (31, 32), qui présente un dispositif de fixation (33) et/ou un dispositif de renvoi (34) pour la corde de traction (20).

9. Bande de compression (100, 150) selon l'une quelconque des revendications précédentes, dans laquelle est associé au corps de base (10) sur une extrémité de sens longitudinal (11, 12) un élément d'enroulement (50) pour serrer et enrouler la corde de traction (20).

10. Auxiliaire orthopédique, auxiliaire médical ou auxiliaire du sport, comprenant une bande de compression (100, 150) selon l'une quelconque des revendications précédentes.

11. Auxiliaire orthopédique selon la revendication 10, dans lequel l'auxiliaire orthopédique est une orthèse (200, 300).

12. Auxiliaire orthopédique selon la revendication 10 ou selon la revendication 11, dans lequel la bande de compression (100, 150) est configurée pour entourer une partie du corps, qui permet une complémentarité circulaire de la bande de compression (100, 150).

13. Orthèse selon la revendication 11 à la revendication 12, dans laquelle l'orthèse (200) ayant pour amortir ou délimiter le mouvement articulé d'une articulation d'extrémité, un manchon (201) entourant l'extrémité en dessous de l'articulation, qui est couplé à une butée (202) pouvant être placée sur l'extrémité au-dessus de l'articulation, dans laquelle s'étend depuis la butée (202) vers le manchon (201) la corde de traction (20), qui est reliée à force à la butée (202) et au manchon (201), dans laquelle le corps de base (10) de la bande de compression (100) est associé au manchon et la bande de compression (100) est réalisée en tant que section d'application de pression du manchon (201), et dans laquelle la corde de traction (20) est configurée de telle manière qu'elle peut être serrée dans l'état placé de l'orthèse (200) par le mouvement articulé de l'extrémité pour ce faisant exercer par l'intermédiaire de la bande de compression (100, 150) une compression sur une zone de partie molle, située en dessous, de l'extrémité afin d'amortir ou de limiter le mouvement articulé.

14. Orthèse selon la revendication 13, dans laquelle la corde de traction (20) peut être serrée par un élément d'enroulement (230) dans la zone de la butée (202).
